# EUROPEAN PATENT SPECIFICATION

(11) **EP 2 251 016 B1**
(45) Date of publication and mention of the grant of the patent: **04.03.2015**
(21) Application number: 09014065.8
(22) Date of filing: 10.11.2009
(51) Int. Cl.: A61K 31/728, A61P 11/04, A61P 11/08, A61P 11/00, A61K 31/19, A61K 36/28

(54) **Topical use of hyaluronic acid with filmogenic action for the treatment and prophylaxys of pathologies of the respiratory tract.**
Topische Verwendung von filmbildendem Hyaluronsäure zur Behandlung und Vorbeugung von Atemwegserkrankungen
Utilisation topique de l'acide hyaluronique filmogène pour le traitement et la prophylaxe des pathologies des voies respiratoires

(30) Priority: 11.11.2008 IT BO20080676
(43) Date of publication of application: 17.11.2010
(73) Proprietor: Farma-Derma S.R.L., 40010 Sala Bolognese (BO) (IT)
(72) Inventor: Russo, Vincenzo, Bologna (IT)
(74) Representative: De Anna, Pier Luigi

(56) References cited:
- WO-A-95/26735
- WO-A-03/096877
- WO-A-2005/000321
- WO-A-2005/067944
- WO-A-2008/009956
- DE-U1- 20 318 634
- FR-A- 2 847 818
- DATABASE WPI Week 200226 Thomson Scientific, London, GB; AN 2002-199323 XP002597318, & JP 2001 346709 A (FANKERU KK) 18 December 2001 (2001-12-18)

## Description

The present invention concerns the realization of a new formulation for topical use with filmogenic action which involves the use of hyaluronic acid associated with chamomile and N-acetylcysteine for the treatment and prophylaxis of pathologies of the upper and lower respiratory tract. The human respiratory system is comprised of nose, mouth (whose main role is to grind food, since it is the first organ of the digestive system, but through it there may be anyway the passage of air and thus the respiration), pharynx and larynx which constitute the upper respiratory tract; and then the trachea which divides into two bronchi which branch into smaller caliber ducts, thus becoming bronchioles and which constitute the lung as a whole the lower respiratory tract.

At this level several pathologies may develop including:
- Tonsillitis: acute or chronic inflammatory process (if it lasts more than three months) which affects the tonsils. It may be caused by viruses or bacteria.
- Pharyngitis: this is an inflammation of the mucosa of the pharynx. It often indicates the beginning of an infection in the upper respiratory tract, for example of a cold or flu. White plaques, red spots and blisters may appear on the mucosa, in addition to redness. The pathological agent usually has a viral origin and it mainly appears in winter, while the bacterial infection is quite rare in this level. There are, instead, chronic and recurring forms of pharyngitis which are frequently related to pathologies affecting other organs or apparatuses, such as in the case of gastroesophageal reflux, which by causing gastric juices to flow back into the pharynx and larynx, it causes the inflammation of these two organs.
- Laryngitis: they are caused by viruses associated with bacteria (pneumococcus, haemophilus Influenthiae). It usually follows a similar process on the upper respiratory tract (rhinitis, adenoiditis, tonsillitis, flu) and it causes dysphonia (reduction of the voice), dry cough, feeling of "dryness" in the throat. The vocal cords are affected by the inflammatory process which mostly damages vocal muscles (myositis) resulting in severe hyposthenia (weakness) which is responsible for the voice reduction. During voice emission, the vocal chords do approach properly on the medial line and there is an air loss. There are chronic forms caused by a continuous inflammation of the larynx due to prolonged exposure to irritant agents including dust, fumes (such as chronic laryngitis due to cigarette smoke), smog, too dry environments, but also the acid content of the stomach which flows back because of gastroesophageal reflux.
- Cough: it is not a real disease but a symptom which starts whenever something irritates the mucosa of throat, trachea or bronchi, which are the canals that carry air into the lungs. It is an automatic defence mechanism that occurs when respiratory tract (large bronchi, pharynx, larynx, trachea) are obstructed by mucus (as in the case of acute bronchitis), as well as by substances or elements coming from outside (dust, smoke, particles suspended in air, badly swallowed objects which end up into respiratory tract) or foreign bodies which forms within the human body (tumours).
- Disorders caused by environmental factors: pollution, smog, dust which are to be found in the air, high temperature and humidity inside the houses, along with passive smoking, represent environmental conditions which are likely to promote the onset of allergies and irritation, but they are not the only ones. The respiratory tract is directly exposed to the harmful action of dust, gases, fumes and vapours which are usually to be found in the environment and it is second only to the skin as target organ for irritants and allergens. Rhinitis occurs with sneezes, rhinorrhoea and nasal obstruction which is often associated with allergic conjunctivitis and involvement of oropharyngeal cavity with ulceration and inflammation of the mucosa.
- Stomatitis is caused by the action of pathogenic microorganisms (viruses, bacteria, fungi) the most common of which are herpes virus, staphylococcus and thrush. It is characterised by an inflammation of the oral mucosa that involves the gums, the inside of the cheeks, the palate, the inside of the lips and tongue. Stomatitis occurs with redness of the mucosa of the mouth, as well as with small ulcerative lesions and sometimes with cankers, small blisters which break and give rise to small white-greyish ulcers which cause pain. Stomatitis may cause the increase of saliva secretion and the swelling of lymph nodes which may be associated with a slight fever. The onset of this disorder may be facilitated by the mechanical action of prostheses, by the use of antiseptic substances and by the intake of antibiotics therapies. Sometimes stomatitis may be the symptom of more important disease: presence of infectious diseases, hormonal disorders, vitamin deficiencies, metabolic disorders, poisoning and immunodeficiency.

WO 95/26735 discloses the use of hyaluronic acid for treating chronic bronchitis, emphysema, and other affections of the respiratory tract, such as asthma, pulmonary edema and acute respiratory distress syndrome per administration via aerosol, by a nebulizer.

DE 203 18 634 U1 describes the use of hyaluronic acid or its salts in a percentage from 0.01-5%, preferably 0.05-1%, more preferably 0.005-0.25%, also in the form of spray, for treating rhinitis.

FR 2 847818 discloses the use of hyaluronic acid in a concentration of 0.01-10 mg/ml (1-1000%) in the form of spray or aerosol, nebulisation, for treating respiratory disorders where an excessive adhesivity of the mucus and epithelial lesions exist, namely rhinitis, bronchitis, mucoviscidosis, bronchiectasis.

WO 03/096877 describes how hyaluronic acid or its sodium salt can interfere in vivo with adhesion, colonization, and disease caused by streptococcal bacteria especially in the pharynx and hence reduce the likelyhood of pharyngitis. Tablets, sprays, aerosols are disclosed.

The present invention is directed to composition comprising hyaluronic acid sodium salt, camomile extract and N-acetylcysteine, in the concentration of hyaluronic acid sodium salt is between 0.01% and 1.0%; camomile extract is between 0.01 % and 5 %; and N-acetylcysteine of between 0.01 % and 9 %, in the formulation and topic use as in independent claims 1, 4, 7 and 9.

The formulation which is object of the invention are proposed in the following types of preparation:
- Spray for oropharyngeal mucosa.
- Aerosol for inhalation therapy.
- Tablets soluble in the mouth for oral mucosa.

It is indicated in the adjuvant treatment and prophylaxis of the above-described diseases including: tonsillitis, laryngitis, pharyngitis, rhinitis, tracheobronchitis and bronchitis, both in the acute and chronic phases; hoarseness; inflammation of vocal cord with or without dysphonia; cough of irritative, post-viral nature or due to environmental pollutants; flu or cold syndromes; after-effects of ENT surgeries; results of extra-oesophageal manifestations due to gastric reflux in the upper respiratory tract (chronic dysphagia, laryngitis and pharyngitis, aphonia, persistent cough). Only the tablets formulation may be used as adjuvant in the treatment of stomatitis and ulcers in the mouth whatever the origin.

The products currently available have several pharmaceutical forms: systemic tablets and syrups; mouthwashes, oral and nasal sprays, tablets and aerosols for topical use. Depending on the included active ingredients, these products carry out different pharmacological activities: NSAIDs (nonsteroidal anti-inflammatory drugs) are used as first-choice in order to relieve symptoms related to the inflammatory process, while corticosteroids (steroidal anti-inflammatory drugs) are administered in more severe cases or, in any case, if the patient suffer from asthma; in both cases, however, these drugs have several side effects, which sometimes affect also the health and safety of patient, thus compromising their use over the time (gastric lesions and many possible alterations of physiological parameters). If the infection is caused by bacteria, an appropriate antibiotic may eradicate it; even in this case the intake of drug would be limited to the time lapse which is necessary to eliminate colonies of bacteria strains embedded in the inflammatory locus (from several days to some weeks).

As shown, all products available on the market have side effects which adversely affect the health of patient and, by evaluating the risk/benefit ratio, they should be used only for those cases where the symptomatology is acute. Whereas, in the cases of chronic or relapsing pathology, it would be advisable to have a product which is effective in reducing the symptoms, and whose long-term use does not compromise the health and safety of patient.

Our study was aimed to the development of an original mixture of ingredients whose combination gives the product a certain complementarity in order to obtain an immediate relief action from disorder of the respiratory tract. In fact, hyaluronic acid, chamomile extract and N-acetylcysteine act synergistically only at topical level, thus carrying out step by step a repairing, cleansing, protective and emollient action with no pharmacological activity.

**Hyaluronic acid** is the main component of a family of polysaccharides containing amino sugars known as glycosaminoglycans, which are the fundamental element of the extracellular matrix. Hyaluronic acid is the main anatomical and functional element of connective tissue and thus it is to be found ubiquitously in most of the tissues and organs, from skin to mucosa. At the endogenous level it retains water in the extracellular matrix thus giving softness and elasticity to the mucosa. Actually, its use as moisturizer is long-standing in cosmetics: in fact, when it is applied to the skin, it restores the proper level of humidity by slowing in a physiological way the process of water evaporation. This humectant action has an affect on the mechanical properties of keratin which becomes more flexible and elastic. Moreover, by depositing on the skin, it forms a protective film which ensures a long-lasting efficacy. When hyaluronic acid is applied on the exterior of the mucosa it has the same properties as on the skin since it presents in any case its chemical and physical characteristics, and therefore it absorbs water by promoting the formation of a film which has the dual function of hydrating and protecting the mucosa. Furthermore, the humid environment created by hyaluronic acid accelerates the process of repair of potential lesions which are to be found on the mucosa due to the inflammation/irritation: in fact, by creating areas where hydration is increased, the cells are less anchored to the extracellular matrix and this allows for a temporary detachment which facilitates the processes of cell migration and division (1, 2) thus promoting the tissue reepithelialisation.

The presence of **chamomile extract** potentiates the effect of hyaluronic acid, because the mucilages of the plant give it a hydrating action and they promote the formation of a mechanical barrier, which put itself on the irritated mucosa thus preventing it from rubbing against other tissues or possible foreign bodies. The importance of forming a protective film when throat is irritated or inflamed, it is particularly relevant since in such conditions, the mucosa is often swollen; this causes a narrowing of the involved cavity, so that the tissues are more easily susceptible to possible friction which may cause discomfort, pain or burning in the patient.

The use of *Chamomilla recutita* as healing herb is widely recognized in the Western world (4). Up to now no adverse reactions have been observed from the use of that plant except for sensitization and allergies which depend from the individual subjects and which can be found in any type of product (5). When applied topically, chamomile has shown to have a remarkable ability to soothe local superficial irritations even when they are associated with oedema, thus decreasing the pain sensation (6) and promoting healing (7) thanks to both the emollient property (due to the presence of mucilages) and antioxidant property (bisabolol and chamazulene).

Finally, the formulation contains **N-acetylcysteine,** a substance whose mucus-cleansing power has been well-known and exploited for long in different areas. Indeed, it gives the medical device a cleansing action towards the oropharyngeal mucosa, which is carried out through a mechanism of depolymerisation of mucoproteinous complexes that consists in the break of disulfide bonds, which chemically make the mucus (8). This allows the viscosity and density of the mucus to decrease and thus it promotes the formation of the protective/repairing barrier. The ability of N-acetylcysteine to degrade bacterial "biofilms" which are often the cause of chronic and/or recurrent inflammations (9), it is a more recent acquisition. The "biofilms" consist of microbial communities of a wide variety of bacterial strains which may also affect the ENT area by reproducing on biological surfaces: bacteria adheres on the mucosa, they reproduce by forming microcolonies and then they begin to produce certain substances which will be of use in forming bacterial biofilm. Therefore, it will consists of bacterial cells which are adhered to the organic surface and included in a large organic extracellular matrix which is mainly composed of polysaccharides and proteins (10). Moreover, under special conditions some bacteria may be released or detach themselves from the biofilm, thus causing symptoms of clinical recrudescence resulting in possible chronic conditions and/or by beginning a new biofilm in sites away from the starting one (11). N-acetylcysteine has shown to be able of interfering with the formation of biofilm in every phase, by acting on bacteria adhesion (first moments), on the synthesis and on the disruption of mature biofilm. In the latter case, the substance acts when the structure is consolidated and therefore it is more difficult to attack and destroy it (12).

Furthermore, N-acetylcysteine carries out a direct antioxidant action, since it has a free thiol group (-SH) that can interact with free radicals which normally reproduce during inflammation states.

The innovation introduced by this invention consists of the combination of synergistic properties related to the substances which constitute the medical device: N-acetylcysteine cleanses the mucosa by taking away mucous secretions and by enabling, on one hand, the emollient activity of chamomile extract and, on the other hand, the hydrating and film-forming activities of hyaluronic acid which will carry out the final function of protecting the mucosa. For these reasons, the medical device can be used for long periods either as prophylaxis of acute inflammation of the upper respiratory tract, or as an adjuvant in the treatment of pathologies which affect the oropharyngeal mucosa, whether acute or chronic.

A particularly preferred example for the spray formulation contains for a solution of 50 ml the following quantities of substances:
- 25 mg hyaluronic acid sodium salt (0.05%)
- 25 mg of chamomile extract (0.05%)
- 250 mg of N-acetylcysteine (0.5%)

A particularly preferred example for the aerosol formulation contains for a solution of 2 ml the following quantities of substances:
- 6 mg of hyaluronic acid sodium salt (0.3%)
- 2 mg of chamomile extract (0.1 %)
- 150 mg of N-acetylcysteine (7.5%)

A particularly preferred example for the formulation of tablets soluble in the mouth contains for a tablet of 1.2 g the following quantities of substances:
- 5 mg of hyaluronic acid sodium salt (0.42%)
- 50 mg of chamomile extract (4.17%)
- 5 mg of N-acetylcysteine (0.42%)

### BIBLIOGRAPHY:

*1.* Beneficial actions of exogenous hyaluronic acid on wound healing. King SR. Hickerson WL, Proctor KG. Surgery 1991 Jan; 109(1):76-84**.**
*2.* Fibronectin and proteoglycan as determinants of cell-substratum adhesion. Culp LA, Murray BA, Rollins BJ. J Supramol Struct 1979;11:401-27.
*3.* Bacteriostatic effects of hyaluronic acid. Pirnazar P, Wolinsky L, Nachnani S, Haake S, Pilloni A, Bernard GW. J Periodontol. 1999 Apr;70(4):370-414**.**
*4.* Matricaria chamomilla (German chamomile) - monograph. No authors listed. Altern Med Rev. 2008 Mar;13(1):58-62**.**
*5.* A review of the bioactivity and potential health benefits of chamomile tea (Matricaria recutita L.). McKay DL, Blumberg JB.Phytother Res. 2006 Jul;20(7):519-30**.**
*6.* Clinical evaluation of fluid extract of Chamomilla recutita for oral aphthae. Ramos-e-Silva M, Ferreira AF, Bibas R, Carneiro S. J Drugs Dermatol. 2006 Jul-Aug;5(7):612-7**.**
*7.* An experimental study of the effects of Matricaria chamomilla extract on cutaneous burn wound healing in albino rats. Jarrahi M. Nat Prod Res. 2008 Mar 20;22(5):422-7
*8.* Bronchial expectorants : pharmacology of bronchial mucomodifiers. Polu JM, Puchelle E, Sadoul P. Sem Hop. 1984 Feb 23;60(9):643-58**.**
*9.* Biofilms on medical devices Talsma SS Home Healthc Nurse. 2007 Oct;25(9):589-94**.**
*10.* Antibiotic resistance of bacteria in biofilms. Curr Opin Otolarynol Head Neck Surg Post JC. 2004; 12: 185-90.
11. Biofilms: survival mechanisms of clinically relevant microorganisms. Clin Microbiol Rev 2002; Donlan RM, Costerton JW. 15: 167-93**.**
12. Inhibitory effect of N-acetylcysteine on adherence of Streptococcus pneumoniae and Haemophilus influenzae to human oropharyngeal epithelial cells in vitro. Riise GC, et al. Respiration 2000; 67: 552-8**.**

## Claims

1. A composition in form of an aerosol formulation comprising hyaluronic acid sodium salt, camomile extract and N-acetylcysteine, in the concentration of:
• hyaluronic acid sodium salt is between 0.01% and 1.0%;
• camomile extract is between 0.01 % and 5 %; and
• N-acetylcysteine of between 0.01 % and 9 %,
for use in treatment of affections of the respiratory tract, wherein the composition is administered at the level of upper respiratory tract.

2. The composition for use of claim 1, wherein the affections of respiratory tract is one or more affections selected from the group of:
oropharyngitis; tonsillitis, laryngitis, pharyngitis, rhinitis, tracheobronchitis and bronchitis, hoarseness and cough of irritative, post-viral nature or due to environmental pollutants; inflammation of vocal cords with or without dysphonia; flu or cold syndromes; side-effects of ORL surgeries; the extraoesophageal manifestations due to gastric reflux in the upper respiratory tract, namely chronic dysphagia, laryngitis and pharyngitis.

3. The composition for use of claims 1 or 2, wherein the composition contains:
• 0.3% of hyaluronic acid sodium salt,
• 0.1 % chamomile extract, and
• 7.5% N-acetylcysteine.

4. A composition in form of a spray formulation comprising hyaluronic acid sodium salt, camomile extract and N-acetylcysteine, in the concentration of:
• hyaluronic acid sodium salt is between 0.01% and 1.0%;
• camomile extract is between 0.01 % and 5 %; and
• N-acetylcysteine of between 0.01 % and 9 %,
for use in treatment of affections of the respiratory tract wherein the composition is administered in form of spray to the oropharyngeal mucosa.

5. The composition for use of claim 4, wherein the affections of the respiratory tract is one or more affections selected from the group of:
oropharyngitis; tonsillitis, laryngitis, pharyngitis, rhinitis, tracheobronchitis and bronchitis, hoarseness and cough of irritative, post-viral nature or due to environmental pollutants; inflammation of vocal cords with or without dysphonia; flu or cold syndromes; side-effects of ORL surgeries; the extreoesophegeal manifestations due to gastric reflux in the upper respiratory tract, namely chronic dysphagia, laryngitis and pharyngitis.

6. The composition for use of claims 4-5, wherein the composition contains:
• 0.05% of hyaluronic acid sodium salt,
• 0.05% chamomile extract, and
• 0.5% N-acetylcysteine.

7. A composition in form of tablets soluble in the mouth comprising hyaluronic acid sodium salt, camomile extract and N-acetylcysteine, in the concentration of:
• hyaluronic acid sodium salt is between 0.01% and 1.0%;
• camomile extract is between 0.01 % and 5 %; and
• N-acetylcysteine of between 0.01 % and 9%,
for use in treatment of affections of the respiratory tract wherein the composition is administered in the mouth to oral mucosa.

8. The composition for use of claim 7, wherein the affections of the respiratory tract is one or more affections selected from the group of:
oropharyngitis; tonsillitis, laryngitis, pharyngitis, rhinitis, tracheobronchitis and bronchitis, hoarseness and cough of irritative, post-viral nature or due to environmental pollutants; inflammation of vocal cords with or without dysphonia; flu or cold syndromes; side-effects of ORL surgeries; the extraoesophageal manifestations due to gastric reflux in the upper respiratory tract, namely chronic dysphagia, laryngitis and pharyngitis.

9. A composition in form of tablets soluble in the mouth comprising hyaluronic acid sodium salt, camomile extract and N-acetylcysteine, in the concentration of:
• hyaluronic acid sodium salt is between 0.01% and 1.0%;
• camomile extract is between 0.01 % and 5 %; and
• N-acetylcysteine of between 0.01 % and 9 %
for use in treatment of stomatitis due to treatments such as chemotherapy and radiotherapy and in case of aphthous ulcers, wherein the composition is administered in the mouth to oropharyngeal mucosa.

10. The composition for use of claim 9, wherein the composition contains:
• 0.42 % of hyaluronic acid sodium salt;
• 4.17% chamomile extract; and
• 0.42% N-acetylcysteine.

## Patentansprüche

1. Zusammensetzung in Form einer Aerosolformulierung, umfassend Hyaluronsäure-Natriumsalz, Kamillenextrakt und N-Acetylcystein in der Konzentration von:
• Hyaluronsäure-Natriumsalz zwischen 0,01 % und 1,0 %;
• Kamillenextrakt zwischen 0,01 % und 5 %; und
• N-Acetylcystein zwischen 0,01 % und 9 %,
zur Verwendung bei der Behandlung von Erkrankungen des Respirationstrakts, wobei die Zusammensetzung auf der Ebene des Respirationstrakts verabreicht wird.

2. Zusammensetzung zur Verwendung nach Anspruch 1, wobei die Erkrankungen des Respirationstrakts eine oder mehrere Erkrankung(en) umfassen, die aus der Gruppe ausgewählt sind mit:
Oropharyngitis; Tonsilitis; Laryngitis; Pharyngitis; Rhinitis; Tracheobronchitis und Bronchitis; Heiserkeit und Husten irritativer, postviraler Natur oder aufgrund von Umweltschadstoffen; Entzündung der Stimmbänder mit oder ohne Dysphonie (Stimmbildungsstörung); Grippe- oder Erkältungssyndromen; Nebenwirkungen von HNO-chirurgischen Eingriffen; extraösophagealen Manifestationen aufgrund von gastrischem Reflux im oberen Respirationstrakt, wie beispielsweise chronischer Dysphagie, Laryngitis und Pharyngitis.

3. Zusammensetzung zur Verwendung nach Anspruch 1 oder 2, wobei die Zusammensetzung enthält:
• 0,3 % Hyaluronsäure-Natriumsalz,
• 0,1 % Kamillenextrakt, und
• 7,5 % N-Acetylcystein.

4. Zusammensetzung in Form einer Sprayformulierung, umfassend Hyaluronsäure-Natriumsalz, Kamillenextrakt und N-Acetylcystein in der Konzentration von:
• Hyaluronsäure-Natriumsalz zwischen 0,01 % und 1,0 %;
• Kamillenextrakt zwischen 0,01 % und 5 %; und
• N-Acetylcystein zwischen 0,01 % und 9 %,
zur Verwendung bei der Behandlung von Erkrankungen des Respirationstrakts, wobei die Zusammensetzung in Form eines Sprays auf die Mund- und Rachenschleimhaut verabreicht wird.

5. Zusammensetzung zur Verwendung nach Anspruch 4, wobei die Erkrankungen des Respirationstrakts eine oder mehrere Erkrankung(en) umfassen, ausgewählt aus der Gruppe mit:
Oropharyngitis; Tonsilitis; Laryngitis; Pharyngitis; Rhinitis; Tracheobronchitis und Bronchitis; Heiserkeit und Husten irritativer, postviraler Natur oder aufgrund von Umweltschadstoffen; Entzündung der Stimmbänder mit oder ohne Dysphonie (Stimmbildungsstörung); Grippe- oder Erkältungssyndromen, Nebenwirkungen von HNO-chirurgischen Eingriffen; extraösophagealen Manifestationen aufgrund von gastrischem Reflux im oberen Respirationstrakt, wie beispielsweise chronischer Dysphagie, Laryngitis und Pharyngitis.

6. Zusammensetzung zur Verwendung nach Ansprüchen 4-5, wobei die Zusammensetzung enthält:
• 0,05 % Hyaluronsäure-Natriumsalz,
• 0,05 % Kamillenextrakt, und
• 0,5 % N-Acetylcystein.

7. Zusammensetzung in Form von Tabletten, die im Mund löslich sind, umfassend Hyaluronsäure-Natriumsalz, Kamillenextrakt und N-Acetylcystein in der Konzentration von:
• Hyaluronsäure-Natriumsalz zwischen 0,01 % und 1,0 %;
• Kamillenextrakt zwischen 0,01 % und 5 %; und
• N-Acetylcystein zwischen 0,01 % und 9 %,
zur Verwendung bei der Behandlung von Erkrankungen des Respirationstrakts, wobei die Zusammensetzung in den Mund auf die Mundschleimhaut verabreicht wird.

8. Zusammensetzung zur Verwendung nach Anspruch 7, wobei die Erkrankungen des Respirationstrakts eine oder mehrere Erkrankung(en) umfassen, ausgewählt aus der Gruppe mit:
Oropharyngitis; Tonsilitis; Laryngitis; Pharyngitis; Rhinitis, Tracheobronchitis und Bronchitis; Heiserkeit und Husten irritativer, postviraler Natur oder aufgrund von Umweltschadstoffen; Entzündung der Stimmbänder mit oder ohne Dysphonie (Stimmbildungsstörung); Grippe- oder Erkältungssyndromen; Nebenwirkungen von HNO-chirurgischen Eingriffen; extraösophagealen Manifestationen aufgrund von gastrischem Reflux im oberen Respirationstrakt, wie beispielsweise chronischer Dysphagie, Laryngitis und Pharyngitis.

9. Zusammensetzung in Form von Tabletten, die im Mund löslich sind, umfassend Hyaluronsäure-Natriumsalz, Kamillenextrakt und N-Acetylcystein in der Konzentration von:
• Hyaluronsäure-Natriumsalz zwischen 0,01 % und 1,0 %;
• Kamillenextrakt zwischen 0,01 % und 5 %; und
• N-Acetylcystein zwischen 0,01 % und 9 %,
zur Verwendung bei der Behandlung von Stomatitis aufgrund von Behandlungen, wie beispielsweise Chemotherapie und Strahlentherapie, und im Falle von Aphthen, wobei die Zusammensetzung in den Mund auf die Mund- und Rachenschleimhaut verabreicht wird.

10. Zusammensetzung zur Verwendung nach Anspruch 9, wobei die Zusammensetzung enthält:
• 0,42 % Hyaluronsäure-Natriumsalz;
• 4,17 % Kamillenextrakt; und
• 0,42 % N-Acetylcystein.

## Revendications

1. Une composition sous la forme d'une formulation d'aérosol comprenant du sel de sodium d'acide hyaluronique, de l'extrait de camomille et de la N-acétylcystéine, dont la concentration est la suivante:
• le sel de sodium d'acide hyaluronique est compris entre 0,01 % et 1,0 %;
• l'extrait de camomille est compris entre 0,01 % et 5 %; et
• la N-acétylcystéine est comprise entre 0,01 % et 9 %,
pour l'utilisation dans le traitement des affections des voies respiratoires, dans laquelle la composition est administrée au niveau des voies respiratoires supérieures.

2. La composition pour l'usage de la revendication 1, dans laquelle les affections des voies respiratoires sont une ou plusieurs des affections sélectionnées parmi le groupe suivant:
oropharyngite; tonsillite, laryngite, pharyngite, rhinite, trachéobronchite et bronchite, enrouement et toux de nature irritative, post-virale ou due à des polluants de l'environnement; inflammation des cordes vocales avec ou sans dysphonie; grippe ou syndromes de refroidissement; effets secondaires de chirurgies ORL; les manifestations extra-oesophagiques dues au reflux gastrique dans les voies respiratoires supérieures, en l'occurrence la dysphasie chronique, la laryngite et la pharyngite.

3. La composition pour l'utilisation des revendications 1 ou 2, cette concentration contenant ce qui suit:
• 0,3 % de sel de sodium d'acide hyaluronique,
• 0,1 % d'extrait de camomille, et
• 7,5 % de N-acétylcystéine.

4. Une composition sous la forme d'une formulation de vaporisation comprenant du sel de sodium d'acide hyaluronique, de l'extrait de camomille et de la N-acétylcystéine; dont la concentration est la suivante:
• le sel de sodium d'acide hyaluronique est compris entre 0,01% et 1,0 %;
• l'extrait de camomille est compris entre 0,01 % et 5 %; et
• la N-acétylcystéine est comprise entre 0,01 % et 9 %,
pour l'utilisation dans le traitement des affections des voies respiratoires, cette composition étant administrée sous la forme de vaporisations sur la muqueuse oropharyngienne.

5. La composition pour l'utilisation de la revendication 4, dans laquelle les affections des voies respiratoires sont une ou plusieurs des affections sélectionnées parmi le groupe suivant:
oropharyngite; tonsillite, laryngite, pharyngite, rhinite, trachéobronchite et bronchite, enrouement et toux de nature irritative, post-virale ou due à des polluants de l'environnement; inflammation des cordes vocales avec ou sans dysphonie; grippe ou syndromes de refroidissement; effets secondaires de chirurgies ORL; les manifestations extra-oesophagiques dus au reflux gastrique dans les voies respiratoires supérieures, en l'occurrence la dysphasie chronique, la laryngite et la pharyngite.

6. La composition pour l'utilisation des revendications 4 et 5, composition qui contient les éléments suivants:
• 0,05 % de sel de sodium d'acide hyaluronique,
• 0,05 % d'extrait de camomille, et
• 0,5% de N-acétylcystéine.

7. Une composition sous forme de comprimés solubles dans la bouche comprenant du sel de sodium d'acide hyaluronique, de l'extrait de camomille et de la N-acétylcystéine, dont la concentration est la suivante:
• le sel de sodium d'acide hyaluronique est compris entre 0,01 % et 1,0 %;
• l'extrait de camomille est comprise entre 0,01 % et 5 %; et
• la N-acétylcystéine est comprise entre 0,01 % et 9 %,
pour l'utilisation dans le traitement des affections des voies respiratoires, cette composition étant administrée dans la bouche sur la muqueuse orale.

8. La composition pour l'utilisation de la revendication 7, dans laquelle les affections des voies respiratoires sont une ou plusieurs affections sélectionnées parmi le groupe suivant:
oropharyngite; tonsillite, laryngite, pharyngite, rhinite, trachéobronchite et bronchite, enrouement et toux de nature irritative, post-virale ou due à des polluants de l'environnement; inflammation des cordes vocales avec ou sans dysphonie; grippe ou syndromes de refroidissement; effets secondaires de chirurgies ORL; manifestations extra-oesophagiques dus au reflux gastrique dans les voies respiratoires supérieures; dysphagie, laryngite et pharyngite chroniques.

9. Une composition sous forme de comprimés solubles dans la bouche comprenant du sel de sodium d'acide hyaluronique, de l'extrait de camomille et de la N-acétylcystéine, dont la concentration est la suivante:
• le sel de sodium d'acide hyaluronique est compris entre 0,01 % et 1,0 %;
• l'extrait de camomille est compris entre 0,01 % et 5 %; et
• la N-acétylcystéine est comprise entre 0,01 % et 9 %,
pour l'utilisation dans le traitement de la stomatite due à des traitements tels que la chimiothérapie et la radiothérapie, et en cas d'ulcères aphteux, dans laquelle la composition est administrée dans la bouche sur la muqueuse oropharyngienne.

10. La composition pour l'utilisation de la revendication 9, dans laquelle la composition contient:
• 0,42 % de sel de sodium d'acide hyaluronique;
• 4,17 % d'extrait de camomille; et
• 0,42 % de N-acétylcystéine.
